# FASCICULE DE BREVET EUROPEEN

(11) **EP 1 733 036 B1**
(45) Date de publication et mention de la délivrance du brevet: **12.03.2014**
(21) Numéro de dépôt: 05746882.9
(22) Date de dépôt: 06.04.2005
(51) Int. Cl.: C12Q 1/68, C12N 15/113, A61K 31/713

(54) **PROCEDE POUR LE PRONOSTIC D'UN neuroblastome**
VERFAHREN ZUR PROGNOSE eines neuroblastom
neuroblastoma PROGNOSIS METHOD

(30) Priorité: 06.04.2004 FR 0450689
(43) Date de publication de la demande: 20.12.2006
(73) Titulaire: BIOMERIEUX D.P.I., 69280 MARCY L'ETOILE (FR); Centre Léon Bérard, 69373 Lyon Cedex 08 (FR)
(72) Inventeur: MOUGIN, Bruno, F-69003 LYON (FR); KRAUSE, Alexander, 38000 Grenoble (FR); PUISIEUX, Alain, F-38300 RUY MONTCEAU (FR); VALSESIA-WITTMANN, Sandrine, CHASSIEU, 69680 (FR)
(74) Mandataire: Bitaud, Valérie Marie-Odile
(86) Numéro de dépôt international: PCT/FR2005/050210
(87) Numéro de publication internationale: WO 2005/096693

(56) Documents cités:
- WO-A-02/059347
- MARTIN T A ET AL: "ELEVATED EXPRESSION OF TRANSCRIPTION REGULATORY PROTEINS TWIST AND SLUG IN HUMAN BREAST CANCER AND THEIR CLINICAL SIGNIFICANCE" BREAST CANCER RESEARCH AND TREATMENT, vol. 82, no. SUPPL 1, 3 décembre 2003 (2003-12-03), pages S117-S118, XP001202653 ISSN: 0167-6806
- WANG X ET AL: "Identification of a novel function of Twist, abHLH protein, in development of acquired taxol resistance in human cancer cells" ONCOGENE, vol. 23, no. 2, 10 janvier 2004 (2004-01-10), pages 474-82, XP002306455 ISSN: 0950-9232
- QADOUMI M ET AL: "IDENTIFICATION OF SIGNATURE GENES FOR LEUKEMIC CTCL CELLS IN S?RY SYNDROME" ARCHIVES OF DERMATOLOGICAL RESEARCH, vol. 295, no. 8/9, 26 février 2004 (2004-02-26), page 380, XP008037396 ISSN: 0340-3696
- YANG J ET AL: "Twist, a master of morphogenesis, plays an essential role in tumor metastasis" CELL, vol. 117, 25 juin 2004 (2004-06-25), pages 927-939, XP002302211 ISSN: 0092-8674
- VALSESIA-WITTMANN SANDRINE ET AL: "Oncogenic cooperation between H-Twist and N-Myc overrides failsafe programs in cancer cells" CANCER CELL, vol. 6, no. 6, décembre 2004 (2004-12), pages 625-630, XP002375775 ISSN: 1535-6108
- POSTIGO ANTONIO A ET AL: "zfh-1, the Drosophila homologue of ZEB, is a transcriptional repressor that regulates somatic myogenesis", MOLECULAR AND CELLULAR BIOLOGY, vol. 19, no. 10, October 1999 (1999-10), pages 7255-7263, ISSN: 0270-7306

## Description

La présente invention concerne le gène twist, et son utilisation dans le domaine du diagnostic et/ou du traitement d'un cancer.

Le cancer est une des plus fortes causes de mortalité dans les pays développés. La détection précoce d'un cancer est essentielle pour prévenir au mieux son développement, le patient a ainsi une meilleure chance de survie et le coût de son traitement s'en trouve potentiellement diminué. Parmi les outils de diagnostic qui permettent de pronostiquer la maladie et d'en faire le suivi, on peut citer l'analyse de l'expression de certains gènes cibles, qui jouent un rôle critique dans l'évolution de certains cancers. Ainsi, dans le cas du cancer du sein, on peut citer le gène cible codant pour le récepteur HER2, qui serait surexprimé dans environ ¼ des cancers du sein invasif (Slamon et al, Science, 1987, 235 :177-182). Dans le cas d'un autre cancer, le neuroblastome, tumeur émergeant des cellules de la crête neurale à l'origine de la formation du tissu nerveux sympathique généralement observé chez l'enfant de moins de cinq ans, le pronostic peut être établi par l'étude de l'oncogène N-myc (Seeger et al, N Engl J Med. 1985 ; 313(18):1111-6 ; Rubie et al J Clin Oncol. 1997 Mar;15(3):1171-82).

Ces gènes cibles sont un atout considérable dans la lutte contre le cancer, car ils peuvent être utilisés comme marqueur de diagnostic et/ou de pronostic du cancer, mais peuvent être également la cible de nouveaux médicaments anticancéreux.

D'une manière surprenante, les inventeurs ont mis en évidence que le gène twist, qui code pour un facteur de transcription bHLH (basic Loop Helix Loop helix) à motif Leucine Zipper, est très pertinent dans la lutte contre le cancer, tel que le neuroblastome. En effet, les inventeurs ont montré que l'inhibition de l'expression du gène Twist permet de restaurer les propriétés apoptotiques des cellules tumorales et la sensibilité à un traitement par chimiothérapie.

Au sens de la présente invention, on entend par cancer, toutes les maladies dues à une multiplication anormale des cellules, notamment de manière non limitatrice, les myélomes, lymphomes, leucémies, carcinomes du rein, du cerveau, du colon, de la prostate, du rectum, du pancréas, des ovaires, du poumon, du foie, du sein, les cancers cutanés choisi parmi les carcinomes, les mélanomes, les neuroblastomes. Selon un mode préféré de réalisation de l'invention, le cancer est un neuroblastome, un mélanome ou un cancer du sein.

Par inhibiteur de l'expression du gène twist, on entend une molécule (ou un ensemble de molécules) qui bloque de manière directe ou indirecte l'expression du gène twist de manière directe ou indirecte, c'est à dire par exemple en bloquant l'expression du gène ou en bloquant l'activité de la protéine codée par ce gène. A titre d'exemple de procédés permettant de bloquer l'expression du gène d'une manière directe, on peut citer : des ARN interférents, des oligonucléotides antisens, ou morpholinos,... A titre d'exemple de procédés permettant de bloquer l'expression du gène d'une manière indirecte , c'est à dire en bloquant l'activité de la protéine, on peut citer : des molécules chimiques interférentes, des aptamères, des mutants dominants négatifs,... Le médicament selon l'invention peut se présenter sous la forme d'une composition pharmaceutique en association avec au moins un excipient pharmaceutiquement acceptable bien connu de l'homme du métier. Dans les compositions pharmaceutiques selon l'invention, pour l'administration orale, sublingale, sous cutanée, intra musculaire, intra veineuse, topique, intratrachéale, rectale, transdermique, l'inhibiteur de l'expression du gène twist peut être administré sous forme unitaire d'administration ou en mélange avec des supports pharmaceutiques classiques, et destinés à une administration par voie orale, par exemple sous la forme d'un comprimé, d'une gélule, d'une solution buvable, etc, ou par voie rectale, sous la forme d'un suppositoire, par voie parentérale, en particulier sous la forme d'une solution injectable, notamment par voie intraveineuse, intradermique, sous cutanée, etc, selon des protocoles classiques bien connus de l'homme du métier.

Lorsqu'on prépare une composition solide sous forme de comprimés, on mélange l'inhibiteur de l'expression du gène twist avec un excipient pharmaceutiquement acceptable également nommé véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées. On peut également les traiter de telles sortes qu'ils aient une activité prolongée ou retardée et qu'ils libèrent d'une façon continue une quantité prédeterminée de l'inhibiteur de l'expression du gène twist. On peut également obtenir une préparation en gélules en mélangeant l'inhibiteur de l'expression de twist avec un diluant et en versant le mélange dans des gélules molles ou dures. On peut également obtenir une préparation sous forme de sirop ou pour l'administration sous forme de gouttes, dans laquelle l'inhibiteur de l'expression du gène twist est présent conjointement avec un édulcorant, un antiseptique, tel que notamment du méthylparaben et du propylparaben, ainsi qu'un agent donnant du goût ou un colorant approprié. Les poudres ou les granules dispersibles dans l'eau peuvent contenir l'inhibiteur de l'expression du gène twist en mélange avec des agents de dispersion ou des agents mouillants, ou des agents de mise en suspension, bien connus de l'homme du métier. Pour une administration parentérale, on utilise des suspensions aqueuses, des solutions salines isotoniques ou des solutions stériles et injectables qui contiennent des agents de dispersion, des mouillants pharmacologiquement compatibles, tels que notamment le propyléneglycol ou le butylèneglycol.

Les inventeurs ont également montré que le gène twist pouvait être utilisé pour le pronostic et/ou diagnostic d'un cancer, tel que notamment le neuroblastome.

A cet effet, l'invention concerne un procédé pour le pronostic et/ou le diagnostic in vitro d'un cancer à partir d'un échantillon biologique prélevé chez un patient selon lequel on détermine l'expression du gène twist.

Selon un mode préféré de réalisation de l'invention, ce procédé comprend les étapes suivantes :
a. on dispose d'un échantillon biologique du patient et on extrait du matériel biologique de l'échantillon biologique
b. on dispose d'au moins un réactif spécifique du gène twist
c. on détermine l'expression du gène twist.

On entend par échantillon biologique, tout matériel issu du patient, susceptible de contenir un matériel biologique permettant de détecter l'expression d'un gène, et peut être notamment un échantillon de sang, de sérum, de salive ou de tissu issus d'un prélèvement de tumeur ou de cellules circulantes du patient. On dispose de cet échantillon biologique par tout type de prélèvement connu de l'homme du métier, tel que notamment une prise de sang ou une biopsie tissulaire.

Selon un mode préféré de réalisation de l'invention, l'échantillon biologique prélevé chez le patient est un échantillon tissulaire ou sanguin.

Tel que défini précédemment, on entend par cancer, toutes les maladies dues à une multiplication anormale des cellules, notamment de manière non limitatrice, les myélomes, lymphomes, leucémies, carcinomes du rein, du cerveau, du colon, de la prostate, du rectum, du pancréas, des ovaires, du poumon, du foie, du sein, les cancers cutanés choisi parmi les carcinomes, les mélanomes, les neuroblastomes. Selon un mode préféré de réalisation de l'invention, le cancer est un neuroblastome. On entend par matériel biologique tout matériel permettant de détecter l'expression d'un gène, comme notamment un acide nucléique ou une protéine. L'acide nucléique peut être notamment un ARN (acide ribonucléique) tel qu'un ARNm (ARN messager).

L'extraction du matériel biologique de l'échantillon biologique lors de l'étape a) peut être réalisée par tous les protocoles d'extraction d'acides nucléiques ou de protéines bien connus de l'homme du métier.

A titre indicatif, l'extraction d'acides nucléique peut notamment être réalisée par une étape de lyse des cellules présentes dans l'échantillon biologique, afin de libérer les acides nucléiques contenus dans les enveloppes protéiques et/ou lipidiques des microorganismes (comme des débris cellulaires qui perturbent les réactions ultérieures). A titre d'exemple, on peut utiliser les méthodes de lyse telles que décrites dans les demandes de brevet WO-A-00/05338 sur la lyse mixte magnétique et mécanique, WO-A-99/53304 sur la lyse électrique, et WO-A-99/15321 sur la lyse mécanique. L'homme du métier pourra utiliser d'autres méthodes de lyse bien connues, telles que les chocs thermiques ou osmotiques ou les lyses chimiques par des agents chaotropiques tels que les sels de guanidium (US-A-5,234,809).

Cette étape de lyse est ensuite généralement suivie d'une étape de purification, permettant la séparation entre les acides nucléiques et les autres constituants cellulaires relargués dans l'étape de lyse. Cette étape permet généralement de concentrer les acides nucléiques. A titre d'exemple, on peut utiliser des particules magnétiques éventuellement revêtues d'oligonucléotides, par adsorption ou covalence (voir à ce sujet les brevets US-A-4,672,040 et US-A-5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrepâ Silica, Promega: MagneSilTM Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-BindTM).

On entend par réactif spécifique un réactif qui réagit avec le matériel biologique afin de mettre en évidence, d'une manière directe ou indirecte l'expression du gène twist, qui peut être déterminé par l'analyse des ARNm issus de ce gène, ou par l'analyse de la protéine codée par ce gène.

A titre indicatif, lorsque l'on souhaite déterminer l'expression du gène twist par l'analyse de la protéine codée par ce gène, ce réactif spécifique comprend au moins un anticorps spécifique de la protéine codée par le gène twist.

A titre indicatif, lorsque l'on souhaite déterminer l'expression du gène twist par l'analyse des ARNm transcrits à partir de ce gène, ce réactif spécifique comprend au moins une amorce d'amplification spécifique de l'ADN complémentaire de cet ARNm (on parle alors d'amorce d'amplification spécifique du gène twist). L'ADN complémentaire d'un ARNm peut être obtenu selon un protocole bien connu de l'homme du métier.

A titre indicatif, on extrait lors de l'étape a) du procédé selon l'invention les ARN totaux (comprenant les ARN ribosomaux, les ARN de transfert, les ARNm) de l'échantillon biologique. On réalise ensuite une réaction de transcription reverse à l'aide d'une enzyme reverse transcriptase qui permet d'obtenir, à partir d'un fragment d'ARN, un fragment complémentaire d'ADN. La réalisation d'une telle étape est bien connue de l'homme du métier. Lorsque l'on souhaite plus particulièrement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription reverse réalisée par l'enzyme reverse transcriptase. On obtient alors différents ADN complémentaires des différents ARN messagers initialement présents dans l'échantillon biologique. Dans la suite de l'exposé, on entend par ADNc, un ADN complémentaire d'un ARN messager.

Par amorce d'amplification, on entend un fragment nucléotidique comprenant de 5 à 100 motifs nucléotidiques, préférentiellement de 15 à 25 motifs nucléotidiques et possédant une spécificité d'hybridation dans des conditions déterminées pour l'initiation d'une polymérisation enzymatique, par exemple dans une réaction d'amplification enzymatique.

Par réaction d'amplification enzymatique, on entend un processus générant de multiples copies d'un fragment nucléotidique cible à l'aide d'amorces d'amplification spécifiques par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US-A-4,683,195, US-A-4,683,202 et US-A-4,800,159,
- LCR (Ligase Chain Reaction), exposée par exemple dans la demande de brevet EP-A-0 201 184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevets US-A-5,399,491.

D'une façon plus précise, la NASBA est une technologie d'amplification isotherme de l'acide nucléique reposant sur l'action conjointe de trois enzymes (transcriptase inverse AMV, Rnase-H et polymérase-ARN T7). Associée à des amorces d'amplification spécifiques d'une séquence cible, elle amplifie les cibles ARN plus d'un milliard de fois en 90 minutes. La réaction d'amplification se produit à 41°C et donne des molécules d'ARN simple brin comme produit final. La NASBA nécessite une paire d'amorce, dont au moins une comprend un promoteur permettant l'initiation de la transcription par une polymérase du bactériophage T7.

On parle d'amplicons pour désigner les polynucléotides générés par une technique d'amplification enzymatique.

Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène twist. Lorsque l'amplification enzymatique est une PCR réalisée après une réaction de transcription reverse, on parle de RT-PCR.

Par sonde d'hybridation, on entend un fragment nucléotidique possédant une spécificité d'hybridation dans des conditions déterminées pour former un complexe d'hybridation avec un fragment nucléotidique cible.

Par hybridation, on entend le processus au cours duquel, dans des conditions appropriées, deux fragments nucléotidiques, tels que par exemple une sonde d'hybridation et un fragment nucléotidique cible, ayant des séquences suffisamment complémentaires sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de là composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par détection on entend soit une détection directe par une méthode physique, soit une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la bétagalactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme 32P, 35S ou 125I. Ainsi, le polynucléotide peut être marqué pendant l'étape d'amplification enzymatique, par exemple en utilisant un nucléotide triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA. Le polynucléotide peut aussi être marqué après l'étape d'amplification, par exemple en hybridant une sonde marquée selon la technique d'hybridation sandwich décrite dans le document WO-A-91/19812.

Un autre mode particulier préférentiel de marquage d'acides nucléiques est décrit dans la demande FR-A-2 780 059. Un autre mode préférentiel de détection utilise l'activité exonucléase 5'-3' d'une polymérase, tel que décrit par Holland P.M., PNAS (1991) p 7276-7280. Des systèmes d'amplification du signal peuvent être utilisés comme décrit dans le document WO-A-95/08000 et, dans ce cas, la réaction préliminaire d'amplification enzymatique peut ne pas être nécessaire.

Au sens de la présente invention, la sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde de détection et le fragment nucléotidique cible marqué.

La sonde d'hybridation peut également être une sonde dite de détection. Dans ce cas, la sonde d'hybridation peut être marquée au moyen d'un marqueur. On réalise alors une réaction d'hybridation entre ladite sonde de capture et le fragment nucléotidique cible. La sonde de détection peut être également une sonde de détection « molecular beacons » telle que décrite par Tyagi & Kramer (Nature biotech, 1996, 14 :303-308).

La détermination de l'expression du gène twist (étape c) peut être réalisée par tous les protocoles connus de l'homme du métier. Cette expression peut être analysée par l'expression des ARNm qui sont transcrits à un moment donné. Dans ce cas, le matériel biologique est un acide nucléique, et le réactif spécifique peut être indifféremment une amorce d'amplification ou une sonde d'hybridation telles que définies précédemment.

L'expression du gène twist peut être également analysé par l'expression des protéines codées par ce gène. Dans ce cas, le matériel biologique est une protéine et le réactif spécifique peut être un anticorps spécifique de la protéine codée par le gène twist. A titre indicatif, on peut déterminer l'expression du gène twist de la manière suivante:
1) après avoir extrait les ARN totaux d'un échantillon biologique tel. que défini lors de l'étape a) du procédé selon l'invention telle que présentée précédemment, on réalise une étape de transcription reverse, telle que décrite précédemment afin d'obtenir les différents ADN complémentaires des différents ARN messagers initialement présents dans l'échantillon biologique (ou ADNc)
2) on amplifie spécifiquement les ADNc. Dans ce cas, le réactif spécifique utilisé comprend au moins une amorce d'amplication spécifique du gène twist. Cette étape peut être réalisée notamment par une réaction d'amplification de type PCR ou par toute autre technique d'amplification telle que définie précédemment.
3) on détermine l'expression du gène twist en quantifiant les ADNc. Les ADNc peuvent être quantifiés notamment par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription reverse, PCR...), on peut normaliser l'expression du gène twist des différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, on corrige ainsi toute variabilité entre les différentes expérimentations. L'homme du métier pourra se référer notamment aux publications suivantes : Bustin SA Journal of molecular endocrinology, 2002, 29: 23-39 ; Giulietti A Methods, 2001, 25 : 386-401.

On peut également déterminer l'expression du gène twist de la manière suivante:
1) après avoir extrait les ARN totaux d'un échantillon biologique tel que défini lors de l'étape a) du procédé selon l'invention telle que présentée précédemment, on réalise une étape de transcription reverse, telle que décrite précédemment afin d'obtenir les différents ADN complémentaires des différents ARN messagers initialement présents dans l'échantillon biologique (ou ADNc)
2) on immobilise les ADNc sur une membrane
3) on détermine l'expression du gène twist en hybridant les ADNc à des sondes d'hybridation spécifiques du gène twist préalablement marquées. De telles techniques d'hybridation sont bien connues de l'homme du métier, et on peut citer notamment la technique du Northern blot. Cette réaction d'hybridation peut être réalisée après une étape d'amplification spécifique des ADN complémentaires des ARN messagers du gène twist.

L'analyse de l'expression du gène twist permet alors de disposer d'un outil pour le diagnostic et / ou le pronostic d'un cancer. On peut par exemple analyser l'expression du gène twist chez un patient susceptible de développer un cancer, et en comparant cette expression avec des valeurs d'expression moyenne connues du gène twist de patients sains et des valeurs d'expression moyenne connues du gène twist de patients atteint d'un cancer, déterminer si le patient développe un cancer afin de lui proposer un traitement adapté.

L'invention concerne enfin un kit de diagnostic et/ou de pronostic d'un cancer comprenant au moins un réactif spécifique du gène twist, tel que défini précédemment.

Les exemples suivants sont donnés à titre illustratif et n'ont aucun caractère limitatif. Ils permettront de mieux comprendre l'invention.

### Exemple 1 - Expression du gène Twist dans le neuroblastome

*1) Caractéristiques des échantillons biologiques :* 23 échantillons de neuroblastome, obtenus auprès du Centre Léon Bérard (CLB) de Lyon, France, ont été utilisées dans cette étude. Ces échantillons de neuroblastome ont été prélevés préalablement à tout traitement thérapeutique. Chaque tumeur a été classée suivant la classification INSS (International Neuroblastoma Staging System; Brodeur et al ; (1993) J. Clin. Oncol. 11, 1466-77). On distinguait alors 12 tumeurs de stade 1/2, 4 tumeurs de stade 4s et 7 échantillons de stade 4 (2 ponctions tumorales, 1 biopsie, 4 ponctions medullaires massivement envahies). L'analyse histochimique montrait dans les tumeurs localisées la présence d'environ 80% de cellules tumorales. L'analyse immunocytochimique montrait également dans les ponctions de moelle osseuse la présence d'environ 80% de cellules tumorales. L'âge médian des patients au moment du diagnostic du neuroblastome était de 10 mois et demi, et 5 patients sont décédés au cours de la période de suivi médian de 75 mois. Les patients ayant décédé au cours de l'étude, et les patients présentant un neuroblatome de stade 4 étaient qualifiés de patients de mauvais pronostic (MP), alors que les patients en vie, ayant développé un neuroblastome de stade 1, 2 et 4s étaient qualifiés de patients de bon pronostic (BP) (qualification selon Brodeur, 2003, Nat Rev Cancer, 203-216). Cette analyse a ainsi été réalisée sur 8 patients MP et 15 patients BP.
*2) Extraction du matériel biologique (ARN totaux) de l'échantillon biologique :* les ARN totaux ont été extraits par l'utilisation d'un tampon Trizol®. Après l'étape d'homogénisation dans 1 à 1,5 ml de tampon Trizol (Invitrogen, Cergy Pointoise, France), les homogénats ont été traités avec 300 µl de chloroforme pour éliminer les contaminants protéiques et lipidiques. Les ARN ont ensuite été précipités avec 750 µl d' isopropanol, et lavés deux fois dans de l'éthanol 80 % (vol/vol) et resuspendus dans de l'eau DEPC. Les ARN totaux ont ensuite été purifiés dans des colonnes Qiagen RNeasy columns (Qiagen, Hilden, Germany) selon les instructions du fabriquant, à l'exception de l'élution finale qui a été réalisée dans 200 µl d'eau RNAse free après une minute d'incubation à 65°C. Avant l'étape de transcription reverse, une étape de précipitation supplémentaire a été réalisée par une solution d'acétate d'ammonium (0,5 volumes, 7,5M) et éthanol (2,5 volumes) afin d'accroître la concentration et la purification des ARN totaux. L'intégralité et la qualité des ARN totaux ont été vérifiées par le bioanalyseur AGILENT 2100 (Agilent Technologies, Waldbronn, Germany).
*3) Hybridation sur puce à ADN :* Afin d'analyser l'expression du gène twist dans le neuroblastomes de patients BP et MP, les ADN complémentaires (ADNc) des ARNm contenus dans les ARN totaux tels que purifiés ci dessus, obtenus à partir de 10 µg d'ARN totaux par l'utilisation de 400 unités de l'enzyme de transcription reverse SuperScriptII (Invitrogen) et 100 pmol d'amorce poly-T contenant le promoteur de la T7 promotor (T7-oligo(dT)24-primer, Proligo, Paris, France). Les ADNc ainsi obtenus ont ensuite été extraits avec du phénol/chloroforme, et précipités tels que décrit précédemment par de l'acétate d'ammonium et de l'éthanol, et remis. en solution dans 24 µl d'eau DEPC. Un volume de 20 µl de cette solution purifiée d'ADNc a fait l'objet ensuite d'une transcription *in vitro* par l'utilisation d'une ARN polymérase T7 qui reconnaît spécifiquement le promoteur de la T7 polymérase tel que mentionné ci dessus. Cette transcription permet d'obtenir l'ARNc de l'ADNc. Cette transcription a été réalisée par l'utilisation d'un kit Bioarray High Yield RNA Transcript Labeling Kit (Enzo Diagnostics, Farmingdale, NY), qui permet non seulement d'obtenir l'ARNc mais également l'incorporation de bases cytidine et uridine biotinylées lors de la synthèse de l'ARNc.

Les ARNc purifiés ont ensuite été quantifiés par spectrophotométrie, et la solution d'ARNc a été ajustée à une concentration de 1 µg/µl d'ARNc. L'étape de clivage de ces ARNc a ensuite été réalisée à 94°C pendant 35 min, par l'utilisation d'un tampon de fragmentation (40 mM de Tris acétate, pH 8,1, 100 mM d'acétate de potassium, 30 mM d'acétate de magnesium) afin de provoquer l'hydrolyse des ARNc et obtenir des fragments de 35 à 200 bp. Le succès d'une telle fragmentation a été vérifié par une électrophorèse sur gel d'agarose 1,5%).

Ensuite, 10 µg d'ARNc fragmentés issus de chaque échantillon ont été ajoutés à un tampon d'hybridation (Affymetrix) et 200 µl de cette solution ont été mis en contact pendant 16 h à 45°C sur une puce d'expression (Human Genome U95Av2 GeneChip® (Affymetrix), selon le protocole d'Affymetrix tel que décrit sur le site internet d'Affymetrix (voir notamment à l'adresse suivante http://www.affymetrix.com/support/downloads/manuals/expression_s2_manual.pdf). Les résultats montraient une surexpression (x 10,8) du gène twist dans les neuroblastomes de patients de mauvais pronostic, par rapport à l'expression du gène twist des neuroblastomes de patients de bons pronostics, indiquant un rôle du gène Twist dans le neuroblastome. L'analyse montrait également une corrélation entre l'expression du gène Twist et l'expression du gène N-Myc.

### Exemple 2 - Corrélation de l'expression du gène Twist et de l'expression du gène N-Myc dans le neuroblastome

*1) Caractéristiques des échantillons biologiques (culture de cellules de neuroblastome):* différentes lignées cellulaires ont été établies par le Centre Léon Bérard à partir de biopsies tumorales et caractérisées quant à leur statut d'amplification du gène *N-Myc,* soit simple copie (CLB-/SedP, -/Ga), soit N-Myc amplifiée (-/Car, -/Pe, -/Esp, -Br, -Bab, -Ber, -/Deb, -Bar, -/Ghe, -Bac, -/Trk, - /LS, -/Ma, -/Rem, -/Vol). Les autres lignées *N-Myc* ont été fournies respectivement par les Dr Bieder, Kemshead, Schwab et l'Institut Gustave Roussy-Paris. Il s'agit respectivement des cellules N-Myc simple copie SK-N-SH, SK-N-AS et SHEP, et N-Myc amplifié WAC, Tet21N et IGR-N91.
Ces lignées cellulaires sont entretenues à 37°C sous 5% de CO2 dans 12 ml de milieu de culture RPMI 1640 (GIBCO Invitrogen corporation) supplémenté en Sérum de Veau Foetal (SVF) décomplémenté 10%, glutamine 2% et pénicilline/streptomycine (10000U/ml; 10000µg/ml). Les cellules sont soumises à l'action de l'EDTA (Life-technologies) et réensemencées à la densité déterminée.
Les cellules MEF ou MEF Imk4a-Arf-/- ont été gracieusement fournies par le Dr M. van Lohuizen. Ces cellules sont des cellules primaires embryonnaires. Ces cellules sont cultivées à 37°C sous 5% de CO2 en milieu DMEM (Invitrogen corporation) supplémenté SVF 10%, glutamine 2% et P/S (10 000 U/ml).
*2) Extraction des ARN :* L'extraction des ARN totaux est réalisée à partir des culots à sec à l'aide du réactif "TriReagent" (Sigma) sur colonne "Phase Lock Gel Heavy" (Eppendorf). Les culots d'ARN obtenus sont resuspendus dans un volume de 20µl d'eau "RNAse-free" et leurs concentrations estimées par lecture de la DO à 260 nm. La synthèse d'ADNc est effectuée avec le "kit First strand cDNA synthesis" (Amersham) à partir de 1 µg d'ARN dilués dans 8µl d'eau "RNAse-free". Les ADNc obtenus sont dénaturés à 95°C pendant 5 min. Les ADNc sont conservés à -30°C jusqu'à leur utilisation.
*3) PCR en temps réel :* Ces résultats ont été confirmés par PCR en temps réel. Pour cela, l'amplification a été effectuée sur l'appareil "ABI-PRISM 7000 Séquence Détection System", dans un volume de 25 µl, en présences des amorces et sondes, présentées ci dessous, à des concentrations respectives de 300 nM, en utilisant le mélange réactionnel "Taq-man Master Mix 2X" (Applied Biosystems) et selon les conditions [95°C pendant 15 sec; 60°C pendant 1 min ] x 50 cycles.
Les amorces et sondes (Eurogentec) utilisées sont rapportées ci-dessous.

| | | |
|---|---|---|
| Amorce N-Myc | 5' | TGATGAAGAG GAAGATGAAC AGG |
| Amorce N-Myc | 3' | TCTTGGGACG CACAGTGATG |
| Sonde N-Myc | Fam-ACTGTGGAGA AGCGGCGTTC CTCCT-Tamra | |
| Amorce Twist | 5' | GGACAAGCTG AGCAAGATTC AGA |
| Amorce Twist | 3' | TCTGGAGGAC CTGGTAGAGG AA |
| Sonde Twist | Fam-AGCTGGCGGC CAGGTACATC GA-Tamra | |
| Amorce ODC | 5' | CTGTCGTCTC AGTGTGAAAT TCG |
| Amorce ODC | 3' | CGCCCGTTCC AAAAGGA |

La spécificité de l'amplicon obtenu a été confirmée par incorporation "SYBR-Green" (Applied Biosystems) sur des dilutions successives au demi des ADNc et obtention d'un pic unique en courbe de dissociation. Les amorces et sondes (marquées VIC en 5' et TAMRA en 3') du gène calibrateur (RNAseP) sont fournies dans le kit "ABI PRISM RNAseP Predevelopped Taq-Man Assay Reagents" (Applied Biosystem). La réaction est effectuée sur 1 µl d'ADN complémentaire, soit 1/90ème du volume de transcription inverse, dilué dans 4 µl d'eau, de façon à standardiser l'étude des différents gènes et minimiser la variabilité des étapes d'extraction d'ARN et de synthèse d'ADNc. Chaque échantillon est testé en deux réplicats.
*4) Northern blot :* Ces résultats ont également été confirmés par Northern blot. Pour cela, 15 µg d'ARN totaux sont séparés sur gel d'agarose 1%, en tampon MOPS-formaldéhyde, sur la nuit à 40 V constant. Le gel est transféré par capillarité sur membrane Hybond N+ (Amersham) pendant une nuit en tampon de transfert (SSC 20x). Les sondes radioactives utilisées sont élaborées en marquage aléatoire à partir du système "Readiprim II" (Amersham) sur 50 ng d'ADN en présence de 5 µCi de dCTP α32. Après 1 heure de marquage à 37°C, la sonde est purifiée sur colonne (mini quick spin DNA columms Roche) et dénaturée pendant 5 min à 95°C avant utilisation. L'hybridation s'effectue en tampon CHURCH (Na2HPO4 1M, NaH2PO4 1M, SDS 7%, BSA 1%) sur la nuit et sous agitation à 65°C. La membrane est lavée avec du SSC 2X pendant 20 min, puis 5 min avec du SSC 0.1X / SDS 0.1% et placée dans une cassette avec un film radiographique à -80°C. Les résultats obtenus confirmaient ceux obtenus sur puce à ADN et sur PCR en temps réel.
*5) Western blot :* Les inventeurs ont également analysé la relation Twist-N myc au niveau protéique par Western blotting, par l'utilisation d'anticorps polyclonaux dirigés contre un épitope C16K correspondant à la partie N-terminale de Twist (Agrobio).

Les culots à sec réalisés sur les lignées cellulaires ont été resuspendus dans 100 à 200 µl de tampon de lyse (Tris HCl pH 7.5 20mM, Triton 1%, SDS 0.05%, Deoxycholate de sodium 0.5%, Nacl 150mM) en présence d'inhibiteur de protéases : cocktail Complete (Roche) et PMSF 0.5% (Roche). Après 30 min d'incubation sur la glace, les échantillons ont été centrifugés à 12000 rpm pendant 5 min à 4°C et le surnageant contenant la fraction protéique a été récupérée. La quantité de protéines totales est estimée par dosage colorimétrique par rapport à une courbe standard d'albumine sérique bovine (0 ; 2 ; 4 ; 8 ; 12 ; 16 ; et 20 µg/ml) et lecture de la densité optique à 595 nm.

Pour l'étude des protéines Twist (27 kDa) et N-Myc (64kDa), 100µg des extraits protéiques totaux, ont été repris dans un volume final de 36 µl en présence de 6 µl de tampon de charge 6x (Tris-HCl (pH 6.8) 375mM, SDS 6%, glycérol 10%, b-mercaptoéthanol 30%, BBP 0.06%) puis déposés sur gel SDS-PAGE 15%. Après migration 2 heures à 100 V constant en tampon Tris-glycine-SDS 1x (SIGMA), le gel a été transféré en condition réfrigérante lh à 200mA sur membrane nitrocellulose de porosité 0,2 µm en tampon Tris-glycine 1x-méthanol 20%.

Après saturation des sites aspécifiques (BSA 3%, lait écrémé 5% ou I-Block 0,2% (Tropix)), les membranes ont été placées en présence de l'anticorps primaire spécifique une nuit à 4°C: (i) anti-N-Myc #556438 (Pharmingen) ou # sc-142 (Tebu) en lait écrémé 0.5%; (ii) anti-Twist (polyclonal de lapin ou de poule anti-C16K Agrobio) en I-Block 0,2%. Après rinçages en TBS-Tween 0.1% (Sigma) les membranes ont été incubées avec l'anticorps secondaire correspondant couplé à la peroxydase HRP (DAKO) 1 heure à température ambiante. Après rinçage, la révélation a été effectuée à l'aide du kit de révélation "ECL" (Amersham) par luminescence et détection sur film Biomax. Les résultats confirmaient au niveau protéique, les résultats obtenus au niveau des ARNm.

*Conclusion : Les inventeurs ont montré que la surexpression du gène Twist était directement associée à la surexpression de N-Myc, que cette dernière soit liée ou non à une amplification génique. Cette association indique que l'expression de Twist est nécessaire pour la survie et la prolifération des cellules présentant une surexpression de N-Myc.*

### Exemple 3 - Le gène Twist est un oncogène impliqué dans l'inhibition de l'apoptose N-Myc dépendante

Sur le plan fonctionnel, les inventeurs ont démontré que la surexpression expérimentale de Twist dans des lignées issues de neuroblastome conduisait les cellules vers un état de sénescence prématurée. Ce type de réponse est un mécanisme de défense cellulaire contre l'induction d'un oncogène (Dimri et al., 1995; pour revue : Mathon and Lloyd, 2001). La sénescence des cellules a été observée par test de coloration X-Gal à pH 6, reflet d'une hyperactivité enzymatique mitochondriale. Les cellules sont rincées avec du PBS 1X, fixées 5 min à température ambiante par une solution PBS 1X, Formaldéhyde 37%, Glutaraldéhyde 25%, et incubées dans la solution de coloration (PBS 1X, K3Fe(CN)6 5mM, K4Fe(CN)6 5mM, MgCl2 2mM, NaCl 150m M, Tampon Phosphate pH6 100mM, C6H5O7Na3 40mM, X-Gal 1mg/ml) à 37°C over-night. Après rinçage à l'eau déminéralisée, la coloration a été évaluée au microscope.

Les inventeurs ont alors montré que c'est la coopération entre H-Twist et N-Myc qui était responsable de la transformation cellulaire, mise en évidence par l'acquisition des cellules à pousser en milieu semi-liquide. Pour cela, les inventeurs ont développé un test de transformation cellulaire de cellules primaires. Deux types de cellules ont été utilisées : des cellules embryonnaires primaires sauvages (MEF wt) mais également des MEF issues de souris portant un KO sur le gène Arf(MEF-InK4a-Arf ^{/-).} Dans ces cellules, le gène InK4a reste fonctionnel mais le gène Arf, régulant positivement p53 est non fonctionnel. Ces cellules ont été gracieusement fournies par le Dr M. van Lohuizen. Ces cellules sont cultivées à 37°C sous 5% de CO2 en milieu DMEM (Invitrogen corporation) supplémenté SVF 10%, glutamine 2% et P/S (10 000 U/ml).

Pour cela, les tests de transformation en soft agar sont effectués sur une base agar à 0,75% (Low melting Point agarase) et un top agar à 0,45% (BMA). Les cellules sont transfectées par la méthode des précipités au phosphate de calcium puis cultivées en triplicat pendant au moins 4 à 6 semaines en présence des antibiotiques ad hoc. Les clones transformants sont numérés et exprimés en % de colonies formées par rapport au nombre de cellules ensemencées.

Dans ce modèle, l'introduction de N-Myc ou H-Twist dans des cellules MEF wt ne permet pas de transformer les cellules mais c'est la coopération entre H-Twist et N-Myc qui confère aux cellules wt la capacité de pousser en milieu semi-liquide, reflet d'une transformation cellulaire. Les inventeurs ont montré que H-Twist interfèrait avec la réponse p53-Arf puisque l'introduction seule de N-Myc provoquait la transformation de MEF-InK4a-Arf^{-/-}, alors que celle de H-Twist non. La surexpression conjointe de N-Myc et de Twist conduisait à une augmentation de la taille et du nombre des colonies transformées, indiquant que H-Twist devait également interférer avec des mécanismes ARF indépendant.

Les inventeurs ont également montré que H-Twist pouvait inhiber la réponse apoptotique induite par N-Myc et que l'inactivation de l'expression de Twist dans des lignées cellulaires N-myc amplifié permettait de restaurer cette apoptose induite par N-Myc. Cette inactivation a été réalisée expérimentalement par interférence ARN ou RNAi, phénomène d'extinction génique post-transcriptionnelle séquence spécifique, induit par l'introduction dans la cellule de petits ARN interférents (siRNA) de 21 à 23 nucléotides (Elbashir et al., 2001).

Pour cela, deux oligonucléotides ARN doubles brins interférents (ARNi) ciblant la région 5' ou 3' de l'ARNm du gène Twist ont été synthétisés. Les séquences des régions cibles reconnues par ces ARNi sont respectivement: AACAGCGAGGAAGAGCCAGAC pour l'ARNi Twist 5', soit les bases 51 à 72 suivant le codon d'initiation de traduction; AAGATGGCAAGCTGCAGCGCTATGT pour l'ARNi Twist3', soit les bases -97 à -76 précédant le codon d'arrêt de traduction. L'ARNi "Scramble", ou aléatoire, de séquence AAGCGCGCTTTGTAGGATTCG, a été choisi pour n'hybrider aucune séquence connue du génome humain.

Les cellules ont été transfectées à l'aide du réactif Exgen 500 par les différents ARNi à une concentration finale de 100 nM, puis l'apoptose a été analysée 72 heures plus tard par cytométrie de flux soit par marquage TUNEL de l'ADN fragmenté à l'aide du kit " In Situ Cell Death Détection Kit, Fluorescein ou TMR" (Roche), soit par évaluation de la sous-population sub-G1 par analyse du cycle cellulaire après incorporation d'iodure de propidium. Dans le premier cas, 10⁶ cellules par point sont fixées par une solution PBS 1X, PAF 1% pendant une heure à température ambiante et perméabilisées à l'aide d'une solution Triton 0,1% en PBS deux minutes sur la glace. Un contrôle positif d'apoptose est effectué par incubation de cellules en présence de DNAse (Boehringer) et un contrôle négatif par incubation des cellules dans le tampon seul. Dans tous les autres puits, un marquage enzymatique dans le tampon est réalisé une heure à 37°C en atmosphère humide et dans le noir. Après deux rinçages PBS, les cellules sont analysées au FACSCAN.

Dans le cas de l'analyse du cycle cellulaire, les cellules sont rincées par 5ml de PBS/BSA 1% à froid puis fixées dans 5ml d'éthanol 70% en vortexant 30 minutes dans la glace. Après rinçage au PBS, les cellules sont perméabilisées par addition de 1 ml d'HCL 2N-triton 0.5% 30 minutes à température ambiante. Après rinçage au PBS, les cellules sont reprises dans 1 ml de PBS contenant 5 µg/ml d'iodure de propidium avant d'être analysées au Facscan. Les résultats obtenus mettaient en évidence que l'inactivation de H-Twist par ARN interférence permettait d'induire jusqu' à 32% de mort cellulaire par rapport aux contrôles non transfectées ou transfectées par un ARN interférent aléatoire qui ne montraient que 4 à 5% de cellules en subG1. Par marquage TUNEL, les inventeurs ont montré que les cellules, dans lesquelles H-Twist était inactivé, mourraient par apoptose.

Les résultats montraient que dans les lignées cellulaires issues de neuroblastomes, l'apoptose N-Myc dépendante était fonctionnelle mais inhibée par la surexpression de H-Twist et qu'il était possible de restaurer la mort cellulaire programmée en inhibant H-Twist.

*Conclusion: l'ensemble de ces résultats démontrent la participation du gène twist dans le développement d'un cancer, tel que le neuroblastome. En particulier, ces résultats démontrent que la possibilité de restaurer la capacité des cellules à mourir par apoptose lorsqu'on bloque l'expression du gène twist, qui est de ce fait un excellent gène cible dans la lutte contre le cancer.*

## Revendications

1. Procédé pour le pronostic d'un neuroblastome comprenant les étapes suivantes :
a. on dispose d'un échantillon biologique du patient et on extrait du matériel biologique de l'échantillon biologique
b. on dispose d'au moins un réactif spécifique du gène twist
c. on détermine l'expression du gène twist.
d. on associe une surexpression du gène twist avec un mauvais pronostic.

## Patentansprüche

1. Verfahren zur Prognose eines Neuroblastoms, umfassend die folgenden Schritte:
a. es steht eine biologische Probe des Patienten zur Verfügung, und es wird biologisches Material der biologischen Probe extrahiert,
b. es steht mindestens ein spezifisches Reagenz für das twist-Gen zur Verfügung,
c. es wird die twist-Gen-Expression bestimmt,
d. es wird eine Überexpression des twist-Gens mit einer schlechten Prognose in Verbindung gebracht.

## Claims

1. Neuroblastoma prognosis method comprising the following steps:
a. biological sample from the patient is provided and biological material is extracted from the biological sample,
b. at least one reagent specific for the twist gene is provided,
c. the expression of the twist gene is determined,
d. an overexpression of the twist gene is associated with a poor prognosis.
